# EUROPEAN PATENT APPLICATION

(11) **EP 1 915 991 A1**
(43) Date of publication of application: **30.04.2008**
(21) Application number: 07020727.9
(22) Date of filing: 23.10.2007
(51) Int. Cl.: A61K 31/05, A61K 31/351, A61K 31/7008, A61P 19/02

(54) **Cartilage regeneration-promoting agent**

(30) Priority: 23.10.2006 JP 2006287232; 29.03.2007 JP 2007086357
(71) Applicant: Eisai Food & Chemical Co., Ltd., Chuo-ku Tokyo (JP)
(72) Inventor: Yoshimura, Hiroyuki, Chuo-ku Tokyo (JP); Koike, Taisuke, Chuo-ku Tokyo (JP)
(74) Representative: Körfer, Thomas

(57) **Abstract**

An object of the present invention is to provide an excellent cartilage regeneration-promoting agent that can promote regeneration of cartilage efficiently and is highly safe, and a medicine and a food that utilizes the cartilage regeneration-promoting agent. Thus, the present invention relates to a cartilage regeneration-promoting agent that includes at least one of hydroxytyrosol and a precursor of hydroxytyrosol, and to a medicine and a food that includes the cartilage regeneration-promoting agent.

## Description

### BACKGROUND OF THE INVENTION

### Field of the Invention

The present invention relates to a cartilage regeneration-promoting agent that promotes regeneration of articular cartilage, and to a medicine and a food that utilize the cartilage regeneration-promoting agent.

### Description of the Related Art

Cartilage covers both ends of bones in the joints and serves as a cushion that absorbs a shock between the bones. In general, the cartilage maintains the normal condition through daily wear and regeneration.

In some cases, however, the cartilage is seriously damaged by, for example, disorders and injuries. In addition, wearing or reduction of resilience of cartilage with aging also makes the cartilage vulnerable to damage. When cartilage is not regenerated fast enough to repair such damages, the function of cartilage is impaired and absorption of a shock between the bones in the joints becomes difficult, resulting in the occurrence of inflammation and pain in the joints.

As for the method for treating or preventing such damages of cartilage, extensive research and development have been done. For example, a method to stimulate biosynthesis of cartilage in the body has been developed that involves administering a composition containing a constituent of cartilage or a precursor thereof to the body orally or through intravenous injection.

More specifically, it is reported that use of glucosamine and chondroitin sulfate, which stimulate production of proteoglycan, a component to retain moisture in articular cartilage and to provide elasticity, or the like, individually or in combination, was effective in the treatment of cartilage injury (See, for example, Japanese Patent No. 2971579). In addition, it is also reported that use of glucosamine and bone marrow extract in combination enhanced the cartilage regeneration-promoting effect of glucosamine (See, for example, Japanese Patent Application Laid-Open (JP-A) No. 2001-322938).

However, there are many patients with cartilage injury due to various reasons e.g. those as described above. Thus, in the present situation, there is a desire to develop a new cartilage regeneration-promoting agent that can promote regeneration of cartilage efficiently and is highly safe.

### BRIEF SUMMARY OF THE INVENTION

An object of the present invention is to solve the conventional problems and to achieve the following objects. Specifically, an object of the present invention is to provide an excellent cartilage regeneration-promoting agent that can promote regeneration of cartilage efficiently and is highly safe, and a medicine and a food that utilizes the cartilage regeneration-promoting agent.

As a result of dedicated investigations conducted by the present inventors to settle the above-mentioned problems, they have found the following experiences or discoveries. Specifically, they have found that hydroxytyrosol, a compound that can be obtained from olive (Family Oleaceae; genus Olea; olive: Olea europaea), has an excellent cartilage regeneration-promoting effect.

The hydroxytyrosol (2-(3,4-Dihydroxyphenyl)ethanol) is a compound represented by the Formula (1) which will be described later, and it has been known that the hydroxytyrosol has inhibitory effect of melanin production and lipid peroxide production, and can be used as a skin external preparation, a bath preparation, or the like (See, for example, JP-A No. 2004-26836). In addition, a method for treating an inflammatory condition such as rheumatoid arthritis by using a treatment agent that contains the hydroxytyrosol and oleuropein is also proposed (See, for example, JP-A No. 2005-517033).

However, it has not been known at all that the hydroxytyrosol has an excellent cartilage regeneration-promoting effect, which is a new finding by the present inventors.

The present invention is based on the above-mentioned experiences or discoveries by the present inventors, and means for solving the above-mentioned problems are as follows. Specifically,
<1> A cartilage regeneration-promoting agent including at least one of hydroxytyrosol and a precursor of hydroxytyrosol.
<2> The cartilage regeneration-promoting agent according to the <1>, wherein at least one of the hydroxytyrosol and the precursor of hydroxytyrosol is derived from olive.
<3> The cartilage regeneration-promoting agent according to one of the <1> and <2>, wherein the precursor of hydroxytyrosol is oleuropein.
<4> The cartilage regeneration-promoting agent according to any one of the <1> to <3>, wherein a content of at least one of the hydroxytyrosol and the precursor of hydroxytyrosol is 10% by mass or more.
<5> The cartilage regeneration-promoting agent according to any one of the <1> to <4>, wherein the cartilage regeneration-promoting agent includes an olive extract.
<6> The cartilage regeneration-promoting agent according to any one of the <1> to <5>, further including an amino sugar in combination with at least one of the hydroxytyrosol and the precursor of hydroxytyrosol.
<7> The cartilage regeneration-promoting agent according to the <6>, wherein the amino sugar is a glucosamine hydrochloride.
<8> The cartilage regeneration-promoting agent according to one of the <6> and <7>, which is a combination agent of at least one of the hydroxytyrosol and the precursor of hydroxytyrosol; and the amino sugar.
<9> The cartilage regeneration-promoting agent according to one of the <6> and <7>, which is a kit that includes a medical agent which contains at least one of the hydroxytyrosol and the precursor of hydroxytyrosol, and a medical agent which contains the amino sugar.
<10> A medicine including the cartilage regeneration-promoting agent of any one of the <1> to <9>.
<11> A food including the cartilage regeneration-promoting agent of any one of the <1> to <9>.
<12> A glycosaminoglycan production-promoting agent including at least one of a hydroxytyrosol and a precursor of hydroxytyrosol.
<13> The glycosaminoglycan production-promoting agent according to the <12>, wherein at least one of the hydroxytyrosol and the precursor of hydroxytyrosol is derived from olive.
<14> The glycosaminoglycan production-promoting agent according to one of the <12> and <13>, wherein the precursor of hydroxytyrosol is oleuropein.
<15> The glycosaminoglycan production-promoting agent according to any one of the <12> to <14>, wherein a content of at least one of the hydroxytyrosol and the precursor of hydroxytyrosol is 10% by mass or more.
<16> The glycosaminoglycan production-promoting agent according to any one of the <12> to <15>, wherein the glycosaminoglycan production-promoting agent includes an olive extract.
<17> The glycosaminoglycan production-promoting agent according to any one of the <12> to <16>, further including an amino sugar in combination with at least one of the hydroxytyrosol and the precursor of hydroxytyrosol.
<18> The glycosaminoglycan production-promoting agent according to the <17>, wherein the amino sugar is a glucosamine hydrochloride.
<19> The glycosaminoglycan production-promoting agent according to one of the <17> and <18>, which is a combination agent of at least one of the hydroxytyrosol and the precursor of hydroxytyrosol; and the amino sugar.
<20> The glycosaminoglycan production-promoting agent according to one of the <17> and <18>, which is a kit that includes a medical agent which contains at least one of the hydroxytyrosol and the precursor of hydroxytyrosol, and a medical agent which contains the amino sugar.
<21> A medicine including the glycosaminoglycan production-promoting agent of any one of the <12> to <20>.
<22> A food including the glycosaminoglycan production-promoting agent of any one of the <12> to <20>.

The present invention can solve the conventional problems and provide an excellent cartilage regeneration-promoting agent that can promote regeneration of cartilage efficiently and is highly safe, and a medicine and a food that utilizes the cartilage regeneration-promoting agent.

### BRIEF DESCRIPTION OF THE SEVERAL VIEWS OF THE DRAWINGS

FIG. 1 shows a cartilage regeneration-promoting effect of hydroxytyrosol for articular cartilage injury of the knee in rabbits.
FIG. 2 is a graph showing a cartilage regeneration-promoting effect of hydroxytyrosol for articular cartilage injury of the knee in rabbits.
FIG. 3 is a graph showing an effect of hydroxytyrosol on muscle weight for articular cartilage injury of the knee in rabbits.
FIG. 4 is tissue staining images (x200) that show histopathologically a cartilage regeneration-promoting effect of hydroxytyrosol for articular cartilage injury of the knee in rabbits.
FIG. 5 is a graph showing an effect of hydroxytyrosol on proteoglycan production for articular cartilage injury of the knee in rabbits.
FIG. 6 is a graph showing an effect of hydroxytyrosol on glycosaminoglycan production for articular cartilage injury of the knee in rabbits.
FIG. 7 shows cartilage regeneration-promoting effects of hydroxytyrosol and glucosamine hydrochloride (for comparison) for articular cartilage injury of the knee in rabbits.
FIG. 8 is a graph showing cartilage regeneration-promoting effects of hydroxytyrosol and glucosamine hydrochloride (for comparison) for articular cartilage injury of the knee in rabbits.
FIG. 9 is a graph showing an effect of hydroxytyrosol and glucosamine hydrochloride (for comparison) on muscle weight for articular cartilage injury of the knee in rabbits.
FIG. 10 is tissue staining images (x200) that show histopathologically cartilage regeneration-promoting effects of hydroxytyrosol and glucosamine hydrochloride (for comparison) for articular cartilage injury of the knee in rabbits.
FIG. 11 is a graph showing effects of hydroxytyrosol and glucosamine hydrochloride (for comparison) on proteoglycan production for articular cartilage injury of the knee in rabbits.
FIG. 12 is a graph showing effects of hydroxytyrosol and glucosamine hydrochloride (for comparison) on glycosaminoglycan production for articular cartilage injury of the knee in rabbits.
FIG. 13 is a graph showing a promoting effect of hydroxytyrosol and glucosamine hydrochloride (combined use) on the production of glycosaminoglycan in rabbit chondrocytes.
FIG. 14 is a graph showing a promoting effect of hydroxytyrosol and glucosamine hydrochloride (combined use) on the production of hyaluronic acid in rabbit chondrocytes.

### DETAILED DESCRIPTION OF THE INVENTION

### (Cartilage regeneration-promoting agent)

The cartilage regeneration-promoting agent of the present invention comprises at least one of hydroxytyrosol and a precursor of hydroxytyrosol (hydroxytyrosol and/or a precursor of hydroxytyrosol) and may further comprise other components according to necessity.

### <Hydroxytyrosol and precursor of hydroxytyrosol>

### -Hydroxytyrosol-

The hydroxytyrosol (2-(3,4-Dihydroxyphenyl)ethanol) is a compound represented by the Formula (1).

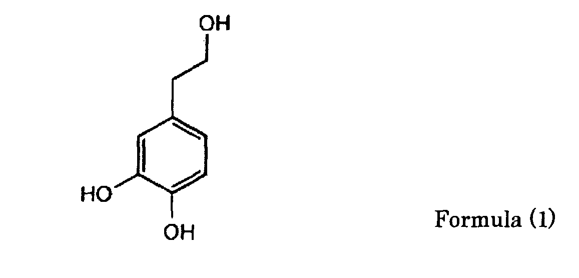

The hydroxytyrosol may be obtained in any manner without limitation, which can be appropriately selected according to the purpose. For example, those chemically synthesized may be used, or those derived from natural products may be used.

When hydroxytyrosol that is derived from natural products is used, the source is not particularly limited and can be appropriately selected according to the purpose; hydroxytyrosol is preferably derived from plants, and more preferably derived from olive (Family Oleaceae; genus Olea; olive: Olea europaea).

The hydroxytyrosol can be obtained, for example, by extraction from a plant body of the olive. Examples of the plant body include leaves, stems, fruits or seeds, and the like.

The hydroxytyrosol can be extracted from the plant body of olive by any method without limitation. The extraction method can be appropriately selected according to the purpose; for example, extraction can be performed using watersoluble organic solvents such as water, methanol, ethanol, and acetone or hydrous organic solvents.

### -Precursor of hydroxytyrosol-

The precursor of hydroxytyrosol refers to a substance that precedes hydroxytyrosol, which is converted to hydroxytyrosol through metabolic reaction within the living body.

Thus, specific examples of the precursor of hydroxytyrosol are not particularly limited and can be appropriately selected according to the purpose. Suitable examples thereof include oleuropein. It is known that when the oleuropein is taken orally, for example, by mammalians such as human and rat, it is hydrolyzed in the body and is converted into hydroxytyrosol (See, Journal of Chromatography B, 785 (2003) 47-56 and Journal of Nutrition, 132: 409-417, 2002). Thus, the precursor of hydroxytyrosol is metabolized to hydroxytyrosol in the body, allowing to achieve a cartilage regeneration-promoting effect similar to that by the hydroxytyrosol.

Like the hydroxytyrosol, the oleuropein may be obtained in any manner without limitation, which can be appropriately selected according to the purpose. For example, those chemically synthesized may be used, or those derived from natural products may be used.

When oleuropein that is derived from natural products is used, the source is not particularly limited and can be appropriately selected according to the purpose; oleuropein is preferably derived from plants, and more preferably derived from olive (Family Oleaceae; genus Olea; olive: Olea europaea).

The oleuropein can be obtained, for example, by extraction from a plant body of the olive. Examples of the plant body include leaves, stems, fruits or seeds, and the like.

The oleuropein can be extracted from the plant body of olive by any method without limitation. The extraction method can be appropriately selected according to the purpose; for example, extraction can be performed with reference to the extraction methods described in JP-A Nos. 2002-128678 and 2003-335693, and the like.

The hydroxytyrosol and the precursor of hydroxytyrosol may be used singly or in combination.

The content of the hydroxytyrosol and/or the precursor of hydroxytyrosol in the cartilage regeneration-promoting agent is not particularly limited, can be appropriately selected according to the purpose, and is preferably 10% by mass or more, more preferably 15% by mass or more, and most preferably 20% by mass or more, based on 100% by mass of the cartilage regeneration-promoting agent. The upper limit of the content is not particularly limited, and with higher content, stronger effect on cartilage regeneration can be expected. The cartilage regeneration-promoting agent may be the hydroxytyrosol and/or the precursor of hydroxytyrosol itself/themselves.

### <Combined use with amino sugar>

The cartilage regeneration-promoting agent may comprise the hydroxytyrosol and/or the precursor of hydroxytyrosol, and an amino sugar in combination or simultaneously.

Such cartilage regeneration-promoting agent that comprises the amino sugar in combination with the hydroxytyrosol and/or the precursor of hydroxytyrosol is advantageous in that the promoting effect on the production of glycosaminoglycan (particularly, hyaluronic acid) in chondrocytes can be enhanced.
By the enhancement of the promoting effect on the production of glycosaminoglycan in chondrocytes, further promotion of cartilage regeneration can be expected.

### -Amino sugar-

The amino sugar is not particularly limited and can be appropriately selected according to the purpose. Examples thereof include glucosamine (2-amino-2-deoxyglucose), mannosamine (2-amino-2-deoxymannose), galactosamine (2-amino-2-deoxygalactose), fucosamine (2-amino-2,6-dideoxygalactose), quinovosamine (2-amino-2,6-dideoxyglucose), rhamnosamine (2-amino-2,6-dideoxymannose), pharmacologically acceptable salts thereof, and the like. In addition, compounds that are these amino sugars with an amino group being acetylated, and pharmacologically acceptable salts of the compounds are also included within the amino sugar. The pharmacologically acceptable salt is not particularly limited and can be appropriately selected according to the purpose. Examples thereof include mineral salts such as a hydrochloride and a sulfate, organic salts such as a citrate, a gluconate, a malate, and an ascorbate. Among these, hydrochlorides are most preferable.

Among the amino sugars, glucosamine, galactosamine, and pharmacologically acceptable salts thereof are preferable, and glucosamine hydrochloride is more preferable.

The amino sugar may be obtained in any manner without limitation, which can be appropriately selected according to the purpose. For example, those chemically synthesized may be used, or those derived from natural products, which can be obtained from carapace of a crab, shrimp, or the like.

The amino sugar may be used singly or in combination.

When the cartilage regeneration-promoting agent comprises the hydroxytyrosol and/or the precursor of hydroxytyrosol and the amino sugar in combination, the content of the amino sugar in the cartilage regeneration-promoting agent is not particularly limited, can be appropriately selected according to the purpose, and is preferably from 10% by mass to 90% by mass, more preferably from 15% by mass to 85% by mass, and most preferably from 20% by mass to 80% by mass.

### -Content ratio-

When the cartilage regeneration-promoting agent comprises the hydroxytyrosol and/or the precursor of hydroxytyrosol, and the amino sugar in combination, the content ratio of the hydroxytyrosol and/or the precursor of hydroxytyrosol, and the amino sugar in the cartilage regeneration-promoting agent is not particularly limited and can be appropriately selected according to the purpose. For example, the hydroxytyrosol and/or the precursor of hydroxytyrosol : the amino sugar is preferably from 20 : 1 to 1 : 100, and more preferably from 1 : 10 to 1 : 70, in terms of mass ratio.

### -Combination agent, and kit-

When the cartilage regeneration-promoting agent comprises the hydroxytyrosol and/or the precursor of hydroxytyrosol, and the amino sugar in combination, the cartilage regeneration-promoting agent may be formed as a combination agent in which the hydroxytyrosol and/or the precursor of hydroxytyrosol, and the amino sugar are mixed. Alternatively, the cartilage regeneration-promoting agent may be prepared as a kit, comprising a medical agent which contains the hydroxytyrosol and/or the precursor of hydroxytyrosol and a medical agent which contains the amino sugar (in which two components mentioned above are not mixed).

### (Medicine)

The medicine of the present invention comprises at least the cartilage regeneration-promoting agent of the present invention and may further comprise other components according to necessity.

The content of the cartilage regeneration-promoting agent in the medicine is not particularly limited and can be appropriately selected depending on, for example, the form of the medicine which will be described later, and a degree of desired effect. The medicine may be the cartilage regeneration-promoting agent itself.

### -Other component-

The other component is not particularly limited and can be appropriately selected according to the purpose unless the effect of the present invention is not impaired. Examples thereof include additives for medicine, which will be described later, and the like. The content of the other components in the medicine is also not particularly limited and can be appropriately selected according to the purpose.

For the above-mentioned medicine, the cartilage regeneration-promoting agent itself, or preparations, obtained by mixing the agent with an appropriate additive, is/are used.

The above-mentioned additive include excipients, binders, lubricants, disintegrants, colorants, flavoring agents, emulsifiers, surfactants, solubilizers, suspending agents, tonicity agents, buffers, antiseptics, antioxidants, stabilizing agents, and absorption promoters, which are used generally for medicine.

Examples of the above-mentioned excipient include lactose, sucrose, glucose, cornstarch, mannitol, sorbitol, starch, pregelatinized starch, dextrin, crystalline cellulose, light anhydrous silicic acid, aluminum silicate, calcium silicate, magnesium aluminometasilicate, calcium hydrogen phosphate, and the like.

Examples of the above-mentioned binder include polyvinyl alcohol, methylcellulose, ethylcellulose, gum arabic, tragacanth, gelatin, shellac, hydroxypropylmethylcellulose, hydroxypropylcellulose, carboxymethylcellulose sodium, polyvinylpyrrolidone, macrogol, and the like.

Examples of the above-mentioned lubricant include magnesium stearate, calcium stearate, sodium stearyl fumarate, talc, polyethylene glycol, colloidal silica, and the like.

Examples of the above-mentioned disintegrant include crystalline cellulose, agar, gelatin, calcium carbonate, sodium hydrogencarbonate, calcium citrate, dextrin, pectin, low-substituted hydroxypropylcellulose, carboxymethylcellulose, carboxymethylcellulose calcium, croscarmellose sodium, carboxymethyl starch, carboxymethyl starch sodium, and the like.

Examples of the above-mentioned colorant include iron sesquioxide, yellow iron sesquioxide, carmine, caramel, beta-carotene, titanium oxide, talc, riboflavin sodium phosphate, yellow aluminum lake, and the like, which are approved as an additive for pharmaceutical agents.

Examples of the above-mentioned flavoring agent include cocoa powder, menthol crystal, aromatic powder, mentha oil, menthol, borneol, powdered cinnamon bark, and the like.

Examples of the above-mentioned emulsifier or the surfactant include stearyl triethanolamine, sodium lauryl sulfate, lauryl aminopropionic acid, lecithin, glycerin monostearate, sucrose fatty acid ester, glycerin fatty acid ester, and the like.

Examples of the above-mentioned solubilizer include polyethylene glycol, propylene glycol, benzyl benzoate, ethanol, cholesterol, triethanolamine, sodium carbonate, sodium citrate, Polysorbate 80, nicotinamide, and the like.

Examples of the above-mentioned suspending agent include, in addition to the surfactants, hydrophilic polymers such as polyvinyl alcohol, polyvinylpyrrolidone, methylcellulose, hydroxymethylcellulose, hydroxyethylcellulose, and hydroxypropylcellulose.

Examples of the above-mentioned tonicity agent include glucose, sodium chloride, mannitol, sorbitol, and the like.

Examples of the above-mentioned buffer include buffers of phosphate, acetate, carbonate, citrate, and the like.

Examples of the above-mentioned antiseptic include methylparaben, propylparaben, chlorobutanol, benzyl alcohol, phenethyl alcohol, dehydroacetic acid, sorbic acid, and the like.

Examples of the above-mentioned antioxidant include sulfite salt, ascorbic acid, alpha-tocopherol, and the like.

Examples of the above-mentioned stabilizing agent include ascorbic acid, disodium edentate, erythorbic acid, tocopherol, and the like.

Examples of the above-mentioned absorption promoter include isopropyl myristate, tocopherol, calciferol, and the like.

The above-mentioned pharmaceutical preparation may include: oral formulation such as tablets, powders, granules, capsules, syrups, troches, and inhalants; external formulation such as suppositories, ointments, ophthalmic ointments, tapes, ophthalmic solutions, nasal drops, ear drops, poultices, and lotions; and injections.

The above-mentioned oral formulation is prepared by appropriately combining the above-mentioned additives. The surfaces of the agents may be coated according to necessity.

The above-mentioned external formulation is prepared by appropriately combining the above-mentioned additives, in particular, excipients, binders, flavoring agents, emulsifiers, surfactants, solubilizers, suspending agents, tonicity agents, antiseptics, antioxidants, stabilizing agents, or absorption promoters.

The above-mentioned injection is prepared by appropriately combining the above-mentioned additives, in particular, emulsifiers, surfactants, solubilizers, suspending agents, tonicity agents, buffers, antiseptics, antioxidants, stabilizing agents, or absorption promoters.

When the cartilage regeneration-promoting agent comprises the hydroxytyrosol and/or the precursor of hydroxytyrosol, and the amino sugar in combination, the medicine may comprise the hydroxytyrosol and/or the precursor of hydroxytyrosol, and the amino sugar simultaneously, or may comprise a medical agent which contains the hydroxytyrosol and/or the precursor of hydroxytyrosol and a medical agent which contains the amino sugar in combination.

Since the medicine comprises at least the cartilage regeneration-promoting agent of the present invention, it has an excellent effect to promote cartilage regeneration. Thus, the medicine is particularly suitably used for the treatment or prevention of cartilage injury.

### (Food)

The food of the present invention comprises at least the cartilage regeneration-promoting agent of the present invention and may further comprise other components according to necessity.

The content of the cartilage regeneration-promoting agent in the food is not particularly limited and can be appropriately selected depending on, for example, the type of the food which will be described later, and a degree of desired effect. The food may be the cartilage regeneration-promoting agent itself.

### -Other component-

The other component is not particularly limited and can be appropriately selected according to the purpose unless the effect of the present invention is not impaired. Examples thereof include a variety of raw materials for food, and the like. The raw material for food is not particularly limited and can be appropriately selected depending on, for example, the type of the food which will be described later. The content of the other components in the food is also not particularly limited and can be appropriately selected according to the purpose. -Type of food-

The type of food is not particularly limited and can be appropriately selected according to the purpose. Examples thereof include confectioneries such jelly, candy, chocolate, and biscuit; luxury drinks such as green tea, tea, coffee, and soft drink; dairy products such as raw milk, yogurt, and ice cream; processed vegetables and fruits such as vegetable drink, fruit drink, and jams; processed meats and fishes such as a ham, sausage, fish sausage, and steamed fish paste; fluid foods such as soup; processed grains such as bread and noodles; and the like. These foods can be produced by any method without limitation and can be appropriately produced, for example, depending on the typical production method of variety of foods.

The food may be produced, for example, as an oral solid formulation such as tablets, granules, and capsules, or as an oral liquid formulation such as drinks and syrups. The production method of the oral solid formulation and the oral liquid formulation is not particularly limited and can be appropriately selected according to the purpose. For example, the oral solid formulation and the oral liquid formulation can be produced in a similar way to the production method of the oral solid formulation and oral liquid formulation of medicine.

When the cartilage regeneration-promoting agent comprises the hydroxytyrosol and/or the precursor of hydroxytyrosol, and the amino sugar in combination, the food may comprise the hydroxytyrosol and/or the precursor of hydroxytyrosol, and the amino sugar simultaneously, or may comprise a food which contains the hydroxytyrosol and/or the precursor of hydroxytyrosol and a food which contains the amino sugar in combination.

Since the food comprises at least the cartilage regeneration-promoting agent of the present invention, it has an excellent effect to promote cartilage regeneration. Thus, the food is particularly useful as a healthy food or a functional food (e.g. supplement) that is taken for the purpose of treatment, improvement, or prevention of cartilage injury.

### [Use]

The cartilage regeneration-promoting agent, the medicine, and the food are suitably applied to patients who have an injury to the cartilage, and the like, and can be used by administering to these patients.

The cartilage regeneration-promoting agent, the medicine, and the food can be administered to any animal without limitation and the animal can be appropriately selected according to the purpose. Examples thereof include human, hamster, dog, cat, horse, cattle, pig, monkey, and the like.

The administration method of the cartilage regeneration-promoting agent, the medicine, and the food is not particularly limited; examples thereof include oral administration, intraperitoneal administration, intravenous administration, intramuscular administration, intraarticular cavity injection, and the like.

The dosage of the cartilage regeneration-promoting agent, the medicine, and the food is not particularly limited and can be appropriately selected depending on: the age and weight of the patient, a subject of administration; a degree of desired effect; and the like. For example, the amount of the hydroxytyrosol and/or the precursor of hydroxytyrosol, as an active ingredient, to be administered to an adult per day is preferably from 0.1 mg/kg weight to 500 mg/kg weight, and more preferably from 1 mg/kg weight to 200 mg/kg weight.

The cartilage regeneration-promoting agent, the medicine, and the food can be administered at any time without limitation, and the time can be appropriately selected according to the purpose. For example, those may be administered prophylactically before cartilage is injured, or may be administered therapeutically after cartilage has been injured.

### [Effect]

The cartilage regeneration-promoting agent, the medicine, and the food have an excellent effect to promote cartilage regeneration. Thus, for example, by administering to the patients with cartilage injury, the cartilage regeneration of the patients can be effectively promoted.

In addition, the cartilage regeneration-promoting agent, the medicine, and the food are also advantageous in that they are highly safe because the active ingredient of them is the hydroxytyrosol and/or the precursor of hydroxytyrosol that can be obtained from natural products such as olive.

### (Glycosaminoglycan production-promoting agent)

It is considered that the cartilage regeneration-promoting agent promotes the production of glycosaminoglycan in chondrocytes and thereby promotes regeneration of cartilage. Thus, the present invention also relates to a glycosaminoglycan production-promoting agent that comprises the hydroxytyrosol and/or the precursor of hydroxytyrosol. In addition, the present invention also relates to a medicine and a food that comprises the glycosaminoglycan production-promoting agent.

Here, the glycosaminoglycan is one of components consisting of cartilage. Specific examples thereof include hyaluronic acid, keratan sulfate, chondroitin, chondroitin sulfate, dermatan sulfate, heparin, heparan sulfate, and the like. The structural form of the chondroitin sulfate is not particularly limited and can be appropriately selected according to the purpose. Examples thereof include chondroitin sulfate A, chondroitin sulfate C, chondroitin sulfate D, chondroitin sulfate E, chondroitin sulfate K, and the like.

Preferably, the glycosaminoglycan production-promoting agent comprises the hydroxytyrosol and/or the precursor of hydroxytyrosol, and the amino sugar in combination. This is advantageous in that the promoting effect on the production of glycosaminoglycan (especially, hyaluronic acid) in chondrocytes can be enhanced.

The details of each component, the content of each component, production method, usage, and the like of the glycosaminoglycan production-promoting agent, and a medicine and a food that contain it are the same as those of the above-mentioned cartilage regeneration-promoting agent, and medicine and food that contain the cartilage regeneration-promoting agent, of the present invention.

### Examples

Hereinafter, Examples of the present invention will be described, which however shall not be construed as limiting the present invention thereto.

### (Production Example 1: Production of hydroxytyrosol)

Hydroxytyrosol according to the present invention was produced in the following way.

100 g of olive leaf was subjected to extraction with 500 mL of 80% hydrous ethanol at a liquid temperature of 50°C for 3 hours. The extraction was performed three times and concentrated under reduced pressure to 200 mL, followed by filtration. pH was adjusted to 2.0 by adding hydrochloric acid to the obtained filtrate, and then acid hydrolysis was performed at a liquid temperature of 50°C for 24 hours. After cooling, the solution was neutralized by adding sodium hydroxide.

Subsequently, the solution was passed over 150 mL of styrene divinylbenzene copolymer resin (Diaion HP-20, manufactured by Mitsubishi Chemical Corporation) at a flow rate of 1.25 mL/min, and the resin was washed with 450 mL of water at a flow rate of 2.5 mL/min. The elution was performed by passing 450 mL of 20% hydrous ethanol at a flow rate of 2.5 mL/min to obtain a solution containing hydroxytyrosol.

This solution was concentrated under reduced pressure to a certain amount to obtain 2.86 g of solid extract that contains 45% of hydroxytyrosol in the concentrated solution.

Next, dextrin was added to adjust the content of hydroxytyrosol to 20%. This solution was freeze dried to obtain 6.43 g of solid (product containing 20% by mass of hydroxytyrosol).

### (Example 1: Investigation (1) of cartilage regeneration-promoting effect of hydroxytyrosol)

The cartilage regeneration-promoting effect of hydroxytyrosol obtained in Production Example 1 was investigated using a rabbit articular cartilage injury model prepared experimentally.

### <Method>

### (1) Habituation

Six female and six male rabbits (New Zealand white, 16 weeks old when introduced, body weight: from 2.00 kg to 2.55 kg) were habituated for one week under the conditions of one rabbit per cage, temperature: from 23°C to 25°C, relative humidity: from 50% to 60%, a light/dark cycle of 12 h, 12 times air changes per hour). During this period, animals were fed with feed, which is sold commercially, and sterile tap water *ad libitum.*

After the period, rabbits were divided at random into two groups so that each group contains three female and three male rabbits. One group was designated as a control group and the other as a hydroxytyrosol administration group (hereinafter, HT group).

After the habituation period, for all rabbits, an articular cartilage injury model was prepared by the method which will be described later.

### (2) Preparation of articular cartilage injury model

The rabbit articular cartilage injury model was prepared based on the method of Hashida et. al. (See, Macromolecular Bioscience, 3, (2003) 596-603), and Tamai et. al. (See, Carbohydrate Polymers, 48, (2002) 369-378, and Carbohydrate Polymers, 54, (2003) 251-262).

Initially, each animal was anesthetized by injecting pentobarbital sodium (40 mg/kg) intraperitoneally. Next, hairs at the knee joint of the left hind limb of the animal were clipped and disinfected with 0.5% chlorhexidine gluconate solution and 70% ethanol solution. Then, an incision was made on the skin on the lateral portion of the knee joint from the central part of the femur toward the tibial tuberosity. The articular capsule was exposed and incised. Further, the knee joint was exposed completely by dislocating the patella toward the medial side. Three holes of 2 mm size in diameter and 4 mm in depth were made by using a hand drill one at the medial trochlear ridge and two at the trochlear sulcus of the femur. The incised portion was washed with sterile saline solution and the articular capsule, and at the same time, the subcutaneous tissues and the skin were sutured with a synthetic absorbent thread and with nylon thread, respectively. During one week after the operation, the sutured portion of the skin was disinfected by povidone-iodine once a day and 10 mg/kg of oxytetracycline hydrochloride was subcutaneously administered twice a day.

### (3) Experiment

After the formation of injury to articular cartilage, for the HT group, the product containing 20% by mass of hydroxytyrosol obtained in Production Example 1 was dissolved in tap water and administered using a water bottle at a rate of 500 mg/kg (100 mg/kg of hydroxytyrosol) per day for 21 days. After making sure that all of the water containing the drug was taken in, rabbits were fed with sterile tap water. The control was fed with sterile tap water *ad libitum.*

### -Observation of general conditions

During the experimental period, diarrhea, appetite, and hair gloss or coat color of each rabbit were observed. In addition, body weight was measured per week.

### -Macroscopic findings of joint region-

After the experimental period, all animals were euthanized by overdose using an intravenous injection of pentobarbital sodium (80 mg/kg). Then, knee joints were incised, and the conditions of joint cartilages were macroscopically observed. The degree, to which the injury of cartilage was healed, was evaluated based on the following standards.

### [Evaluation Standards]

- : Degree of healing is less than 50%
+ : Degree of healing is from 50% to 60%
++ : Degree of healing is from 60% to 80%
+++ : Degree of healing is from 80% to 100%

Further, the above evaluation was assigned numbers: (-) was assigned with 0 point, (+) was assigned with 1 point, (++) was assigned with 2 points, and (+++) was assigned with 3 points. The degree of healing of articular cartilage was expressed numerically, and Mann-Whitney's U test was performed between the control group and the HT group.

### -Measurement of muscle weight-

For each animal, left and right lateral vastus muscles and femoral biceps muscles were collected, and weights were measured. The muscle weight ratio was calculated by comparing the side, in which cartilage injury was made, with the opposite side, and Student's t-test was performed between the control group and the HT group.

### -Histopathological examination-

For each animal, femur was collected and fixed by a 10% neutral buffered formalin solution. Then, the injured portion, normal portion, and growth zone (epiphyseal line) of knee articular cartilage were trimmed to a thickness of 5 mm as a specimen and decalcified by being shaken in 5% formic acid solution for 24 hours. The specimen was neutralized in a 5% sodium sulfate solution for 24 hours and washed for 10 hours under running water. The specimen was embedded in paraffin according to usual method and was sliced by a microtome into 5 µm slices. Then, each specimen was subjected to hematoxylin/eosin staining and histopathologically examined.

### -Image analysis-

The specimens stained with Safranin O and with Alcian blue were prepared in the same way as mentioned above and subjected to image analysis. The 200 times magnified images of specimens were scanned into the computer using a digital camera (Olympus DP70, manufactured by Olympus Corporation). Then, two locations (20,000 pixels) were randomly sampled, and the density of the site stained in a specific color (site stained with Safranin O: corresponding to proteoglycan, site stained with Alcian blue: corresponding to glycosaminoglycan) was measured. For the obtained values, Student's t-test was performed between the control group and the HT group.

### <Results>

### -General conditions-

For both the HT group and the control group, marked change was not observed in general conditions (diarrhea, appetite, and hair gloss or coat color, etc). In addition, with respect to body weight change during test period, significant difference was not observed between the HT group and the control group (data not shown).

### -Macroscopic findings of joint region-

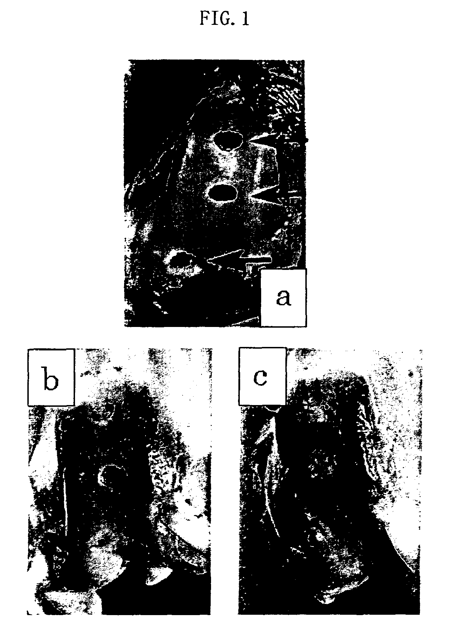

FIG. 1-(a) shows the appearance of knee joint immediately after making cartilage injury. The arrows indicate from the top, a proximal hole in trochlear sulcus, distal hole in trochlear sulcus, and hole in medial trochlear ridge.

FIG. 1-(b) shows the knee joint of the control group after the experimental period. The control group did not show complete healing of cartilage injury. FIG. 1-(c) shows the knee joint of the HT group after the experimental period. Administration of hydroxytyrosol promoted the regeneration of cartilage.

Table 1 below is a table summarizing the results of macroscopic observation of each individual where the degree of healing of cartilage injury was observed at 3 weeks after operation. The evaluation standards of - to +++ are as described above.

**Table 1**

| | | Degree of healing | | |
|---|---|---|---|---|
| | Sex | Medial trochlear ridge | Proximal trochlear sulcus | Distal trochlear sulcus |
| Control group | | | | |
| 1 | Female | + | ++ | + |
| 2 | Female | +++ | + | +++ |
| 3 | Female | ++ | ++ | + |
| 4 | Male | ++ | ++ | + |
| 5 | Male | + | + | + |
| 6 | Male | ++ | ++ | + |

| HT group | | | | |
|---|---|---|---|---|
| 1 | Female | +++ | ++ | +++ |
| 2 | Female | +++ | +++ | +++ |
| 3 | Female | ++ | + | + |
| 4 | Male | +++ | ++ | ++ |
| 5 | Male | + | +++ | ++ |
| 6 | Male | +++ | +++ | +++ |

The above results were expressed in numerical value and compared. Compared with the control group, in the HT group, cartilage healing was promoted significantly (p<0.05).

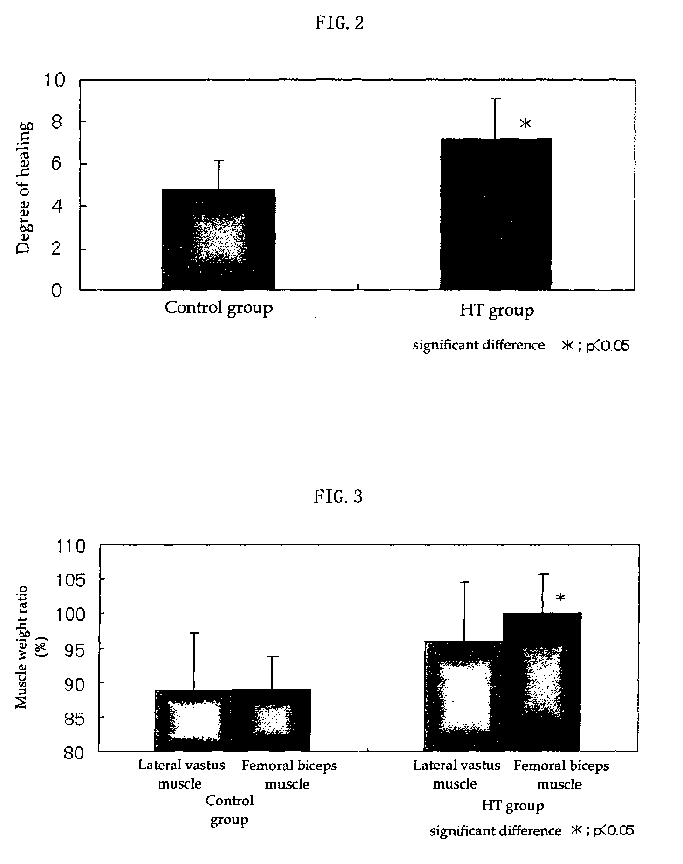

In addition, while the average value of quantified data was 4.83 for the control group, that of the HT group was 7.17 (FIG. 2).

### -Muscle weight-

The calculated weight ratios of the lateral vastus muscle and the femoral biceps muscle are shown in Table 2 and comparison between the control group and the HT group is shown in FIG. 3.

**Table 2**

| | | Muscle weight ratio (%) | |
|---|---|---|---|
| | Sex | Lateral vastus muscle | Femoral biceps muscle |
| Control group | | | |
| 1 | Female | 76.61 | 88.56 |
| 2 | Female | 90.91 | 89.50 |
| 3 | Female | 93.24 | 87.30 |
| 4 | Male | 80.89 | 90.92 |
| 5 | Male | 98.93 | 81.31 |
| 6 | Male | 92.48 | 96.32 |
| Mean | | 88.84 | 88.99 |
| Standard deviation | | 8.38 | 4.89 |

| HT group | | | |
|---|---|---|---|
| 1 | Female | 105.30 | 100.18 |
| 2 | Female | 96.92 | 98.45 |
| 3 | Female | 80.89 | 93.46 |
| 4 | Male | 93.29 | 94.80 |
| 5 | Male | 95.10 | 106.36 |
| 6 | Male | 104.10 | 107.22 |
| Mean | | 95.93 | 100.08 |
| Standard deviation | | 8.82 | 5.74* |

In the control group, marked decrease in the muscle weights of the lateral vastus muscle and femoral biceps muscle was observed. In contrast, in the HT group, the muscle weight of the lateral vastus muscle decreased slightly, but little decrease in the muscle weight of the femoral biceps muscle was found.

### -Histopathological examination-

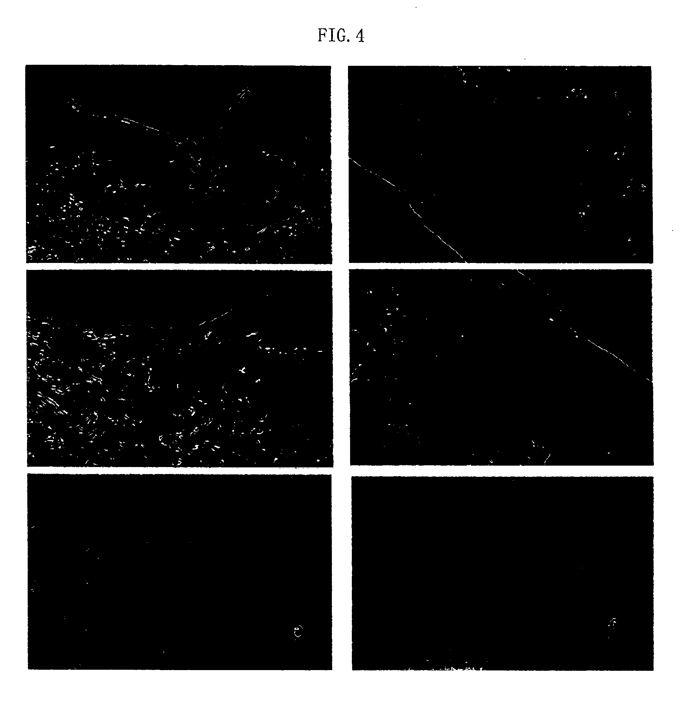

FIG. 4 shows histopathological images of the control group and of the HT group; hematoxylin/eosin staining images (FIG. 4-(a) and 4-(b)), Safranin O staining images (FIG. 4-(c) and 4-(d)), and Alcian blue staining images (FIG. 4-(e) and 4-(f)) of the site at which injury was created. (a), (c), and (e) in the left column of FIG. 4, and (b), (d), and (f) in the right column of FIG. 4 correspond to the images of the control group and those of the HT group, respectively.

In the control group, infiltration of macrophages and osteoclasts, and proliferation of capillary vessels and fibroblasts were found at the site at which injury was created, but few neoplastic chondrocytes were found.

On the other hand, in the HT group, there was little connective tissue, and matured chondrocytes were found extensively. Since in the images, proliferation of osteoclasts and capillary vessels was not observed, it is speculated that the regeneration of cartilaginous tissue had almost finished.

### -Image analysis-

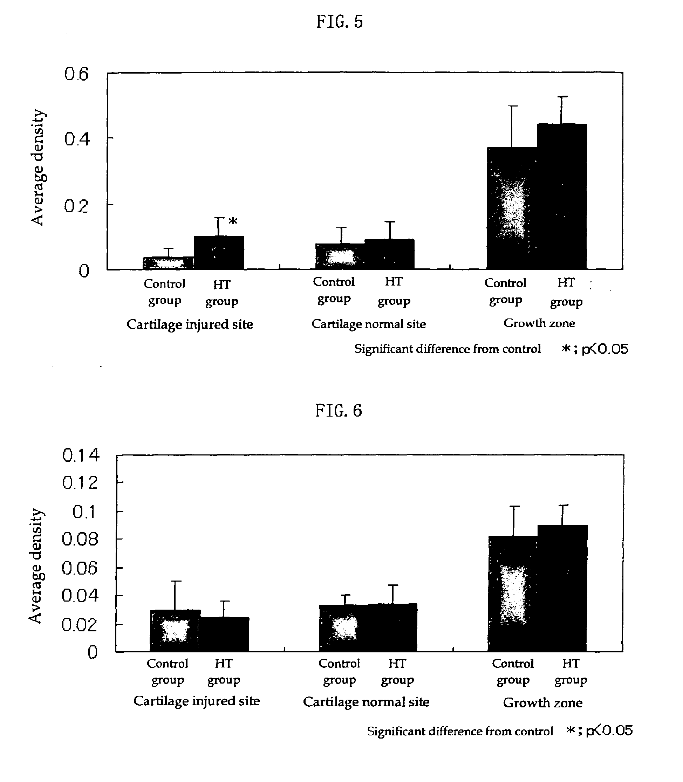

Results of image analysis of the control group and the HT group are shown in FIGS. 5 and 6. FIG. 5 is a result of image analysis of Safranin O staining, and FIG. 6 is a result of image analysis of Alcian blue staining.

At the site of cartilage at which injury was created, the HT group showed a significant increase of the density at the site stained with Safranin O in comparison with the control group. This reveals that in the HT group, production of proteoglycan was promoted. In contrast, at the normal site and growth zone of cartilage, there were no difference between the control and the HT group (FIG. 5).

### (Example 2: Investigation (2) of cartilage regeneration-promoting effect of hydroxytyrosol)

In the same way as in Example 1, the cartilage regeneration-promoting effects were investigated with respect to hydroxytyrosol (100 mg/kg per day, HT group) and with respect to glucosamine hydrochloride (800 mg/kg per day, hereinafter, GAH group) for comparison. The control group was set in the same way as in Example 1.

### <Results>

### -Observation of general conditions-

For any of the control, HT and GAH groups, change in general conditions was not observed. Body weight changes both of the HT group and of the GAH group were almost the same as that of the control group.

### -Macroscopic findings of joint region-

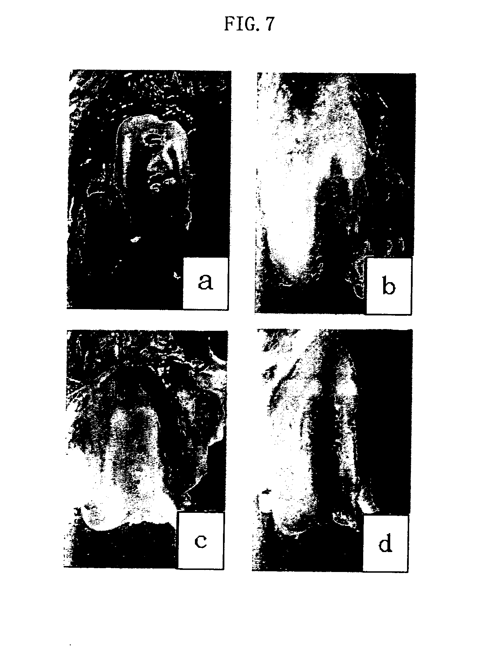

FIG. 7-(a) shows the appearance of knee joint immediately after making cartilage injury.

FIG. 7-(b) shows the knee joint of the control group after the experimental period. The control group did not show complete healing of cartilage injury. FIG. 7-(c) and 7-(d) show the knee joints after the experimental period of the HT group and the GAH group, respectively. Administration of hydroxytyrosol or glucosamine hydrochloride promoted the regeneration of cartilage.

Table 3 below is a table, as in Example 1, that summarizes the results of macroscopic observation of each individual where the degree of healing of cartilage injury was observed at 3 weeks after operation.

**Table 3**

| | | | Degree of healing | |
|---|---|---|---|---|
| | Sex | Medial trochlear ridge | Proximal trochlear sulcus | Distal trochlear sulcus |
| Control group | | | | |
| 1 | Female | + | + | ++ |
| 2 | Female | ++ | + | +++ |
| 3 | Female | ++ | ++ | +++ |
| 4 | Male | ++ | + | + |
| 5 | Male | ++ | + | +++ |
| 6 | Male | ++ | + | ++ |

| HT group | | | | |
|---|---|---|---|---|
| 1 | Female | +++ | +++ | +++ |
| 2 | Female | +++ | +++ | +++ |
| 3 | Female | +++ | ++ | +++ |
| 4 | Male | ++ | ++ | +++ |
| 5 | Male | +++ | +++ | +++ |
| 6 | Male | ++ | ++ | +++ |

| GAH group | | | | |
|---|---|---|---|---|
| 1 | Female | +++ | ++ | ++ |
| 2 | Female | +++ | +++ | +++ |
| 3 | Female | +++ | +++ | +++ |
| 4 | Male | +++ | + | ++ |
| 5 | Male | +++ | + | + |
| 6 | Male | ++ | +++ | ++ |

The above results were expressed in numerical value and compared. Compared with the control group, cartilage healing was promoted significantly both in the HT group (p<0.01) and in the GAH group (p<0.05).

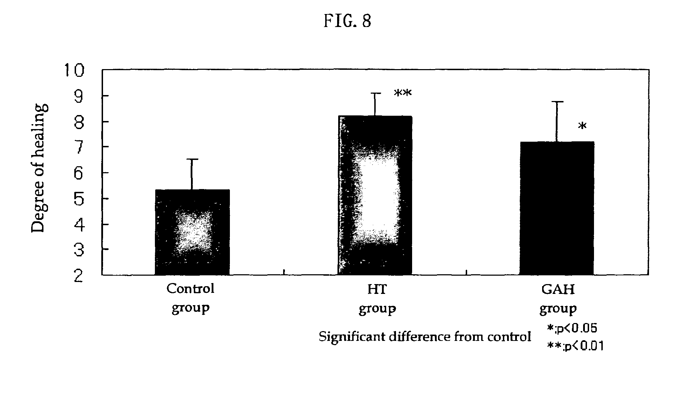

In addition, while the average value of quantified data was 5.33 for the control group, it was 8.17 for the HT group and 7.17 for the GAH group. The cartilage regeneration was promoted more in the HT group than in the GAH group (FIG. 8).

### -Muscle weight-

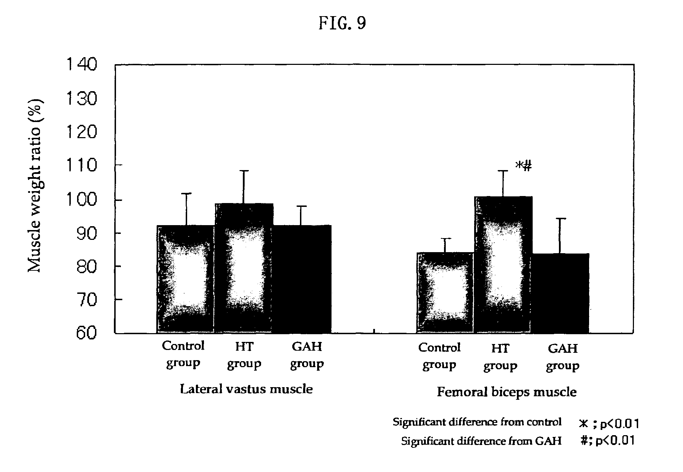

The calculated weight ratios of the lateral vastus muscle and the femoral biceps muscle are shown in Table 4 and comparison among the control group, the HT group, and the GAH group is shown in FIG.9.

**Table 4**

| | | Muscle weight ratio (%) | |
|---|---|---|---|
| | Sex | Lateral vastus muscle | Femoral biceps muscle |
| Control group | | | |
| 1 | Female | 74.09 | 81.31 |
| 2 | Female | 91.69 | 86.96 |
| 3 | Female | 101.70 | 76.81 |
| 4 | Male | 100.20 | 90.57 |
| 5 | Male | 92.87 | 83.67 |
| 6 | Male | 90.92 | 83.48 |
| Mean | | 91.91 | 83.80 |
| Standard deviation | | 9.84 | 4.71 |

| HT group | | | |
|---|---|---|---|
| 1 | Female | 94.98 | 90.91 |
| 2 | Female | 98.56 | 111.86 |
| 3 | Female | 92.43 | 93.42 |
| 4 | Male | 84.93 | 101.37 |
| 5 | Male | 111.10 | 104.66 |
| 6 | Male | 108.90 | 101.31 |
| Mean | | 98.48 | 100.59 |
| Standard deviation | | 10.00 | 7.61* |

| GAH group | | | |
|---|---|---|---|
| 1 | Female | 82.24 | 78.13 |
| 2 | Female | 96.46 | 86.21 |
| 3 | Female | 90.85 | 65.10 |
| 4 | Male | 98.02 | 92.37 |
| 5 | Male | 89.59 | 86.12 |
| 6 | Male | 94.99 | 93.81 |
| Mean | | 92.03 | 83.62 |
| Standard deviation | | 5.79 | 10.64 |

In the control group and the GAH group, marked decrease especially in the muscle weight of femoral biceps muscle was observed. For the lateral vastus muscle, slight decrease in the muscle weight was observed. In contrast, in the HT group, little decrease in the muscle weights both of lateral vastus muscle and of femoral biceps muscle was found.

### -Histopathological examination-

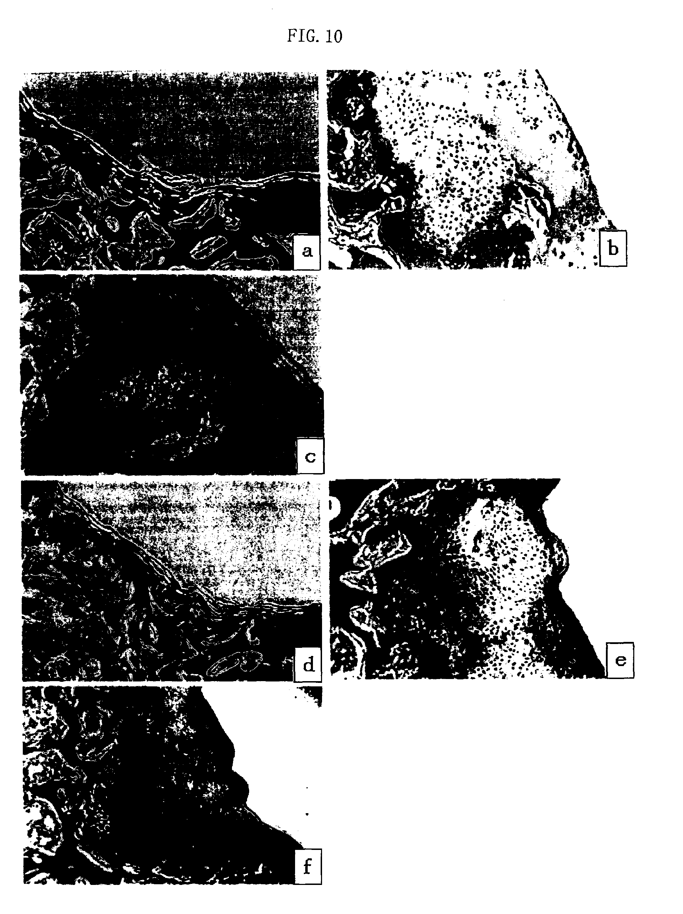

FIG. 10 shows histopathological images of the control group, the HT group, and the GAH group; hematoxylin/eosin staining images (FIG. 10-(a) to 10-(c)), and Safranin O staining images (FIG. 10-(d) to 10-(f)). FIG. 10-(a) and 10-(d) show the images of the control group, FIG. 10-(b) and 10-(e) the images of the GAH group, and FIG. 10-(c) and 10-(f) the images of the HT group.

In the control group, infiltration of macrophages and osteoclasts, and proliferation of capillary vessels and fibroblasts were found at the site at which injury was created, but few neoplastic chondrocytes were found.

On the other hand, in the HT group, there was little connective tissue, and matured chondrocytes were found extensively. Since in the images, proliferation of osteoclasts and capillary vessels was not observed, it is speculated that the regeneration of cartilaginous tissue had almost finished.

In the GAH group, almost the same findings as those of the HT group were observed.

### -Image analysis-

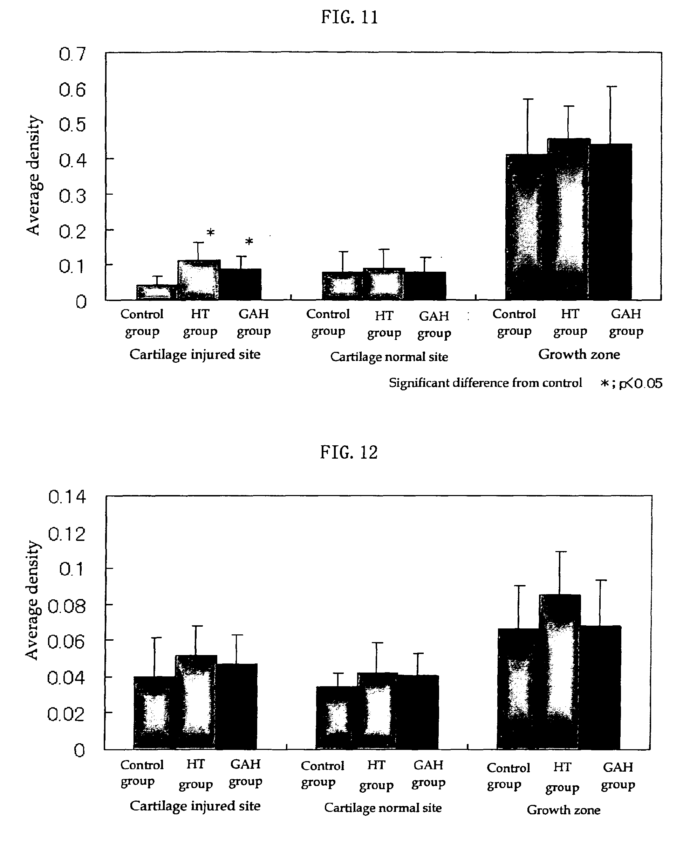

Results of image analysis of the control, HT, and GAH groups are shown in FIGS. 11 and 12. FIG. 11 is a result of image analysis of Safranin O staining, and FIG. 12 is a result of image analysis of Alcian blue staining.

The HT group and the GAH group showed a significant increase of the density at the site stained with Safranin O in the injured site of cartilage in comparison with the control group. This reveals that in the HT group and the GAH group, production of proteoglycan was promoted. The production of proteoglycan was promoted more in the HT group than in the GAH group. In contrast, at the normal site and growth zone of cartilage, there were no difference among the control group, the HT group, and the GAH group (FIG. 11).

The above results of Examples 1 and 2 demonstrated that the cartilage regeneration-promoting agent of the present invention has an effect to promote healing of cartilage injury (cartilage regeneration). Since harmful effects of the cartilage regeneration-promoting agent according to the present invention on the general conditions were not observed, safety of the cartilage regeneration-promoting agent of the present invention was also confirmed.

### (Example 3: Combined effect of hydroxytyrosol and glucosamine hydrochloride)

The promoting effects of hydroxytyrosol and glucosamine hydrochloride on the production of glycosaminoglycan and on the production of hyaluronic acid in chondrocytes were evaluated.

Rabbit chondrocytes (rabbit articular chondrocytes in primary culture, F-8, Cell Garage Co.,Ltd.) were washed with phosphate buffered saline solution, and then cells were released with 0.05% trypsin-EDTA solution.

The released chondrocytes were cultured in DMEM medium containing 10% FBS, 100 U/ml penicillin, 100 µg/ml streptomycin, 100 µM trisodium ascorbyl-2-phosphate (available from SIGMA-ALDRICH Corp.), and 25 mM HEPES (available from Invitrogen Corporation) at 37°C in a humidified condition with 5% CO₂.

The above-mentioned chondrocytes were plated on a 24-well plate coated with collagen at 2.5 x 10⁵ cells per well (1 ml/well), and after cultivation for 7 days, the medium was removed by aspiration.

Then, to the above-mentioned cultured chondrocytes, was added 2 ml/well of the above-mentioned medium in which hydroxytyrosol (purity: 98%, available from Cayman Chemical) at a final concentration of 100 ng/ml (hereinafter, HT group), glucosamine hydrochloride (purity: 99%, available from Tokyo Health Foods Ltd.) at a final concentration of 4 µg/ml (hereinafter, GAH group), or both the above hydroxytyrosol and the above glucosamine hydrochloride (hereinafter, HT+GAH group) was/were mixed and dissolved, and further cultured for 12 days.

As a control group, a group was set in which cells were cultured without adding chemicals.

### -Determination of number of viable cells and measurement of each amount produced-

For each group after 12 days culture, the number of viable cells was determined by WST assay.

The percentage of the amount of glycosaminoglycan or hyaluronic acid produced by each experimental group, relative to the control group, was calculated according to the following formula: ((amount produced by each experimental group/ number of cells of each experimental group) x 100)/ (amount produced by the control group/ number of cells of the control group).

### 1) Measurement of amount of glycosaminoglycan produced

The amount of glycosaminoglycan produced by the above-mentioned cultured cells was measured using a measurement kit (acid mucopolysaccharide quantification kit, available from Cell Garage Co.,Ltd.).

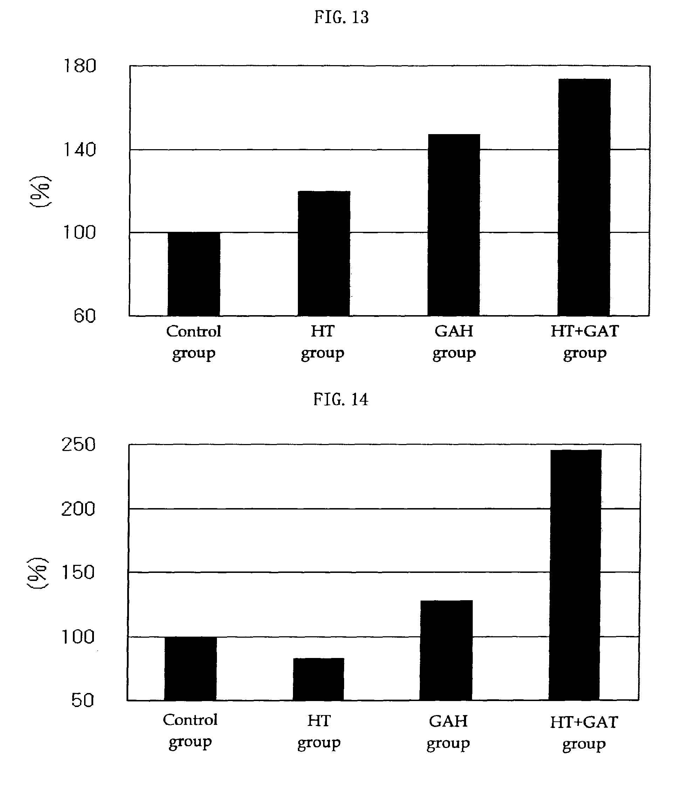

As a result, the amounts of glycosaminoglycan produced by the HT group and the GAH group were 119.8% and 147.0%, respectively, compared to the control group. In addition, the amount produced by the HT+GAH group was 173.4% compared to the control group, revealing that use of hydroxytyrosol and glucosamine hydrochloride in combination generates a synergistic effect (FIG. 13).

### 2) Measurement of amount of hyaluronic acid produced

The supernatant of cultured cells of each group was collected, and the amount of hyaluronic acid produced was measured using a measurement kit (hyaluronic acid measurement kit, available from Seikagaku Corporation).

As a result, the amounts of hyaluronic acid produced by the HT group and the GAH group were 82.9°/ and 127.4%, respectively, compared to the control group. In addition, the amount produced by the HT+GAH group was 245.2% compared to the control group, revealing that use of hydroxytyrosol and glucosamine hydrochloride in combination generates a synergistic effect (FIG. 14).

The above-mentioned results of Example 3 demonstrated that the cartilage regeneration-promoting agent of the present invention has an effect to promote production of glycosaminoglycan in chondrocytes, and that particularly in an aspect where both hydroxytyrosol and glucosamine hydrochloride are used in combination, the effects (among others, promoting effect on the production of hyaluronic acid) are enhanced synergistically.

The cartilage regeneration-promoting agent, medicine, and food of the present invention are particularly suitable, for example, for the application to the patients with cartilage injury.

## Claims

1. A cartilage regeneration-promoting agent comprising
at least one of hydroxytyrosol and a precursor of hydroxytyrosol.

2. The cartilage regeneration-promoting agent according to claim 1, wherein at least one of the hydroxytyrosol and the precursor of hydroxytyrosol is derived from olive.

3. The cartilage regeneration-promoting agent according to one of claims 1 and 2, wherein the precursor of hydroxytyrosol is oleuropein.

4. The cartilage regeneration-promoting agent according to any one of claims 1 to 3, further comprising an amino sugar in combination with at least one of the hydroxytyrosol and the precursor of hydroxytyrosol.

5. The cartilage regeneration-promoting agent according to claim 4, wherein the amino sugar is a glucosamine hydrochloride.

6. A medicine comprising the cartilage regeneration-promoting agent of any one of claims 1 to 5.

7. A food comprising the cartilage regeneration-promoting agent of any one of claims 1 to 5.

8. A use of at least one of hydroxytyrosol and a precursor of hydroxytyrosol for the production of a cartilage regeneration-promoting agent.
